# EUROPÄISCHE PATENTSCHRIFT

(11) **EP 1 003 499 B1**
(45) Veröffentlichungstag und Bekanntmachung des Hinweises auf die Patenterteilung: **26.09.2001**
(21) Anmeldenummer: 98934939.4
(22) Anmeldetag: 09.06.1998
(51) Int. Cl.: A61K 31/19, A61K 9/12, A61K 47/10, A61K 47/22

(54) **ZUR TOPISCHEN APPLIKATION BESTIMMTE DICLOFENAC-LÖSUNG**
DICLOFENAC SOLUTION FOR TOPICAL APPLICATION
SOLUTION DE DICLOFENAC POUR APPLICATION TOPIQUE

(30) Priorität: 17.06.1997 DE 19725615
(43) Veröffentlichungstag der Anmeldung: 31.05.2000
(73) Patentinhaber: Permamed AG, 4106 Therwil (CH)
(72) Erfinder: LUTZ, Christian, CH-4143 Dornach (CH)
(74) Vertreter: Huber, Bernhard, Dipl.-Chem.
(86) Internationale Anmeldenummer: EP9803457
(87) Internationale Veröffentlichungsnummer: WO9857624

(56) Entgegenhaltungen:
- EP-A- 0 129 285
- EP-A- 0 245 126
- EP-A- 0 491 076

## Beschreibung

Die Erfindung betrifft eine Arzneimittelzubereitung zur topischen Anwendung, die Diclofenac als Wirkstoff enthält.

Diclofenac ([2-(2,6-Dichloranilino-)phenyl]essigsäure, C₁₄H₁₁Cl₂NO₂) isteine antiphlogistisch und antirheumatisch wirksame Verbindung, die zur Behandlung von Schmerzen, Rheuma und Entzündungen angewendet wird. Diclofenac wird der Gruppe der nichtsteroidalen Antirheumatika (NSAR) zugerechnet und weist entzündungshemmende, analgetische und antipyretische Eigenschaften auf, welche auf die Hemmung der Biosynthese von Prostaglandinen zurückgeführt werden.

Bekannte Formulierungen von Diclofenac, die zur topischen Anwendung geeignet sind, umfassen Gele und Pflaster. Solche Formulierungen basieren im Allgemeinen auf bekannten Lösungsmitteln und Penetrationsverstärkern, wie z.B. Propylenglykol und Alkohol. Ein Beispiel für ein im Handel erhältliches Diclofenac-Präparat in Form eines Gels zur topischen Anwendung ist Voltaren® Emulgel, das den Wirkstoff Diclofenac-Diethylamin sowie als Trägerstoffe ein Acrylsäurepolymerisat, Capryl/Caprinsäure, Cetomakrogol, Diethylamin, 2-Propanol, Paraffin und Propylenglykol enthält. Ein Beispiel für ein Präparat in Form eines Pflasters ist Flector® EP Tissue Gel, das den Wirkstoff Diclofenac-Epolamin sowie Propylenglykol als Träger enthält. Ein weiteres zur Verwendung in einem Pflaster geeignetes Diclofenac-Präparat wird in der DE-A 37 20 896 beschrieben.

Die bisher bekannten topischen Anwendungsformen von Diclofenac weisen jedoch erhebliche Nachteile auf. So ist das Aufbringen des Wirkstoffs mittels Pflaster im Kopf-Hals-Bereich aus praktischen und ästhetischen Gründen nicht möglich, obwohl Diclofenac gerade zur Behandlung von Beschwerden im Kopf-Hals-Bereich geeignet ist. Auch die Anwendung von Gelen mit einer langen Verweildauer auf der Haut, ist im Kopf-Hals-Bereich aus praktischen Gründen nicht durchführbar. Zur Behandlung von Beschwerden im Kopf-Hals-Bereich kommen deshalb bisher Diclofenacenthaltende Präparate zur Anwendung, die oral eingenommen werden. Die orale Einnahme von Diclofenac ist jedoch, insbesondere bei einer Langzeittherapie mit ernsten Nebenwirkungen bis hin zu tödlichen Folgen, verbunden. Aufgrund der schwerwiegenden Nebenwirkungen ist eine Limitierung der systemischen Konzentration auf ein möglichst niedriges Niveau vor allem für chronisch Kranke notwendig.

Die Verwendung von Trägerstoffen und Penetrationsverstärkern in zur topischen Anwendung vorgesehenen Präparaten ist grundsätzlich bekannt. Die Wirkungsweise und Eignung von Penetrationsverstärkern ist jedoch zumeist auf bestimmte Wirkstoffe beschränkt. So beschreiben Sasaki et al., (J.Pharm.Pharmakol., 72 (1990) 196 bis 199) eine Erhöhung der transdermalen Penetration von Phenolsulfonphthalein durch Zugabe von 1-Methyl-2-pyrrolidon und 1-Lauryl-2-pyrrolidon ( = N-Dodecyl-2-pyrrolidon). Eine Verbesserung der transdermalen Penetration anderer Wirkstoffe, insbesondere von Diclofenac, wird von den Autoren jedoch nicht in Betracht gezogen. Vielmehr lässt sich anderen Berichten der gleichen Autoren (Sasaki et al., J.Pharmacobio-Dyn., 13 (1990) 200 bis 205; Sasaki et al., Chem.Pharm. Bull., 38 (8) (1990) 2308 bis 2310) entnehmen, dass der Effekt der Penetrationsverstärkung durch 1-Methyl-2-pyrrolidon oder 1-Lauryl-2-pyrrolidon wirkstoffabhängig ist und dass zudem bei Verwendung von 1-Lauryl-2-pyrrolidon alleine Hautreizungen beobachtet wurden.

Eine Aufgabe der vorliegenden Erfindung war es, eine Arzneimittelzubereitung zur topischen Anwendung bereitzustellen, die es ermöglicht, Diclofenac direkt aus einer Lösung aufzutragen und die ein hohes Maß an lokaler Verträglichkeit und Wirksamkeit bei gleichzeitiger Minimierung der unerwünschten systemischen Verfügbarkeit und Wirksamkeit bereitstellt. Diese Aufgabe wird erfindungsgemäß gelöst durch eine Arzneimittelzubereitung zur topischen Anwendung, enthaltend Diclofenac als Wirkstoff, welche dadurch gekennzeichnet ist, dass der Wirkstoff in einem Lösungsmittelgemisch gelöst vorliegt, welches mindestens einen Alkylalkohol mit 2 bis 4 C-Atomen als Hauptbestandteil, mindestens ein kurzkettiges N-Alkylpyrrolidon mit 1 bis 4 C-Atomen im Alkylrest und mindestens ein mit einem langkettigen Alkylrest, der 10 bis 16 C-Atome aufweist, substituiertes Pyrrolidon enthält.

Bei der erfindungsgemäßen Arzneimittelzubereitung handelt es sich um eine echte Lösung, sodass Diclofenac mit 100 % relativer Bioverfügbarkeit (per Definition) bei der Anwendung zur Verfügung steht. Da es sich um eine echte Lösung des Wirkstoffs in einer Penetrationsvermittler enthaltenden Lösung handelt, findet eine sofortige Penetration in die Haut statt, sodass kein bzw. nur ein unsichtbarer Verbleib der Formulierung auf der Haut vorliegt. Das Aufbringen der erfindungsgemäßen Lösung ermöglicht eine hohe lokale Wirkung unter maximaler Ausnutzung der pharmakokinetischphysiologischen Grundvoraussetzungen einer lokalen Arzneimitteltherapie. Die Bereitstellung von Diclofenac in Lösung stellt zudem eine leicht anwendbare, hygienische und reproduzierbare Darreichungsform dar. Die erfindungsgemäße flüssige Arzneimittelzubereitung kann auf übliche Weise aufgetragen werden. Besonders vorteilhaft sind Darreichungsformen in Form eines Dosieraerosols oder in Form eines Stifts zum Aufrollen. Durch einen Austausch der Aufsätze ist dabei eine duale Anwendung möglich. Ein Dosieraerosol ist insbesondere für großflächiges Aufsprühen, z.B. auf den Rücken bei Lumbago/Hexenschuß zur Verringerung der systemischen Belastung des Gastrointestinaltraktes geeignet. Ein Stift zum Aufrollen findet insbesondere im Kopfbereich, wie etwa bei Zahnschmerzen oder Kopfschmerzen Anwendung, da hier aufgrund einer möglichen Augenreizung die Verwendung eines Sprays nachteilig sein kann. Gerade bei Schmerzen im Kopfbereich, wie etwa Zahnschmerzen oder Kopfschmerzen ist das Aufbringen von Diclofenac aus einer echten Lösung, z.B. mittels eines Stiftes zum Aufrollen, pharmakokinetisch ideal, da die beste lokale Resorption des Wirkstoffes an der Kinnlade erreicht wird. Dies führt zu einer erheblichen Verringerung der benötigten Wirkstoffkonzentration und somit zu einer deutlichen Herabsetzung der unerwünschten Nebenwirkungen. Auch für Asthmatiker oder Allergiker ist die Anwendung der erfindungsgemäßen Arzneimittelzubereitung in Form eines Stiftes zum Aufrollen gegenüber einem Spray vorzuziehen, da dadurch die Gefahr eines akuten Anfalls vermieden werden kann. Auch für alte Patienten, vor allem für Rheumatiker mit krankheitsbedingter Deformation der Gelenke kann die leicht zu handhabende Darreichungsform als Stift zum Aufrollen gegenüber einem Spray vorteilhaft sein. Sowohl mit einem Dosieraerosol als auch mit einem Stift zum Aufrollen ist eine reproduzierbare Dosierung der erfindungsgemäßen Diclofenac enthaltenden Arzneimittelzubereitung möglich.

Überraschenderweise wurde festgestellt, dass die erfindungsgemäße Diclofenac enthaltende Arzneimittelzubereitung, in der der Wirkstoff in Lösung vorliegt, wesentliche Vorteile gegenüber den bisher bekannten Arzneimittelzubereitungen in Form eines Gels oder Pflasters aufweist. So konnten bei Versuchen an Zellkulturen mit menschlicher Haut erheblich höhere Wirkstoffkonzentrationen im Sinne einer wesentlichen Penetrationsverstärkung und Bioverfügbarkeit nachgewiesen werden. Während therapeutisch mit etwa 2/3 der Menge der erfindungsgemäßen Arzneimittelzubereitung die gleiche Wirkung wie mit einem herkömmlichen Gel erzielt werden konnte, wurde gleichzeitig toxikologisch kein wesentlicher Unterschied festgestellt. Dies bedeutet, dass durch Verwendung der erfindungsgemäßen Arzneimittelzubereitung die Wirkstoffdosis und damit die unerwünschten Nebenwirkungen beträchtlich verringert werden können, wobei gleichzeitig eine leicht anwendbare, hygienische und reproduzierbare Darreichungsform von Diclofenac in Form einer echten Lösung bereitgestellt wird.

Die erfindungsgemäße Diclofenac als Wirkstoff enthaltende Arzneimittelzubereitung umfasst einen Alkylalkohol mit 2 bis 4 C-Atomen als Hauptbestandteil. Geeignete Alkylalkohole sind Ethanol, n-Propanol, iso-Propanol, n-Butanol, iso-Butanol sowie tert.-Butanol. Bevorzugt ist der Alkylalkohol Ethanol oder Propanol und besonders bevorzugt Ethanol. Weiterhin umfasst das Lösungsmittelgemisch ein kurzkettiges N-Alkylpyrrolidon. Das kurzkettige N-Alkylpyrrolidon weist 1 bis 4 C-Atome auf. Geeignete N-Alkylpyrrolidone sind N-Methylpyrrolidon, N-Ethylpyrrolidon, N-Propylpyrrolidon, N-lso-Propylpyrrolidon, N-Butylpyrrolidon, N-iso-Butylpyrrolidon und N-tert.-Butylpyrrolidon. Besonders bevorzugt umfasst das Lösungsmittelgemisch N-Methylpyrrolidon.

Neben dem kurzkettigen N-Alkylpyrrolidon umfasst das Lösungsmittelgemisch mindestens ein mit einem langkettigen Alkylrest substituiertes Pyrrolidon. Das langkettig substituierte Alkylpyrrolidon weist dabei 10 bis 16 C-Atome im Alkylrest auf. Besonders bevorzugt ist Laurylpyrrolidon (1-Lauryl-2-pyrrolidon).

In der erfindungsgemäßen Arzneimittelzubereitung beträgt der Wirkstoffgehalt bevorzugt 0,5 bis 5 % Gewicht, bezogen auf das Gewicht der gesamten Arzneimittelzubereitung, mehr bevorzugt von 0,5 bis 2 % und am meisten bevorzugt 0,5 bis 1,5 %. (Alle Prozentangaben hierin beziehen sich auf das Gewicht, falls nicht anders angegeben.)

Grundsätzlich können das kurzkettige N-Alkylpyrrolidon und das mit einem langkettigen Alkylrest substituierte Pyrrolidon in beliebigen Verhältnissen vorliegen. Bevorzugt liegen das kurzkettige N-Alkylpyrrolidon und das langkettige Alkylpyrrolidon im Molverhältnis von 10 bis 100:1, besonders bevorzugt von 20 bis 40:1 vor. Weiterhin umfasst die Arzneimittelzubereitung bevorzugt von 50 bis 80 Vol.-% Alkohol, bezogen auf das Gesamtvolumen der Arzneimittelzubereitung. Besonders bevorzugt ist eine Zubereitung, welche 65 bis 75 Gew.-% Ethanol, 5 bis 20 Gew.-% N-Methylpyrrolidon und 0,3 bis 2 Gew.-% 1-Lauryl-2-pyrrolidon, jeweils bezogen auf das Gesamtvolumen der Arzneimittelzubereitung enthält.

Die erfindungsgemäße Arzneimittelzubereitung ist zur dermalen Arzneimitteltherapie beim Menschen vorgesehen. Die topische Applikation soll dabei zu ausschließlicher lokaler Wirkung am Ort der Anwendung führen, wobei eine systemische Wirkung als unerwünschte Wirkung so weit wie möglich auszuschalten ist. Mit der erfindungsgemäßen Diclofenac-Lösung kann ein therapeutisches Verhältnis von hoher lokaler Wirkung und guter lokaler Verträglichkeit bei einer gleichzeitigen Minimierung der unerwünschten systemischen Verfügbarkeit und systemischen Wirkung erhalten werden. Ziel einer Diclofenac-enthaltenden Formulierung ist zwar grundsätzlich die Steigerung der Penetration. Der Bereich des therapeutischen Nutzens ist jedoch eng begrenzt durch das notwendige Äquilibrium aus Penetrationsverstärkung und Limitierung von Toxizität. Überraschenderweise ist es mit der erfindungsgemäßen Arzneimittelzubereitung möglich, durch indirekte Wirkungsverstärkung die Wirkstoffdosis zu reduzieren, wobei gleichzeitig die lokale Wirkung erhalten bleibt und die systemische Wirkung und Toxizität nicht erhöht wird.

Die erfindungsgemäße Arzneimittelzusammensetzung kann weitere pharmazeutisch geeignete Hilfs- und Trägerstoffe sowie Wasser umfassen. So umfasst eine beispielhafte, bevorzugte Lösung je 100 g 0,5 bis 5 g Diclofenac, 1,0 bis 5,0 g Dexpanthenol, 1,0 bis 5,0 g Diisopropyl-adipat und/oder Diisopropylpalmitat und/oder Diisopropylstearat, 0,1 bis 3 g Polyvinylpyrrolidon (PVP), 5 bis 20 g N-Methyl-2-pyrrolidon, 0,3 bis 2 g 1-Lauryl-2-pyrrolidon, 65 bis 75 g Ethanol 96 % und als Rest Wasser. Eine besonders bevorzugte Zusammensetzung umfasst je 100 g Lösung 0,5 bis 2 g Diclofenac, 1,0 bis 2,0 g Dexpanthenol, 2,0 bis 3,0 g Diisopropyladipat und/oder Diisopropylpalmitat und/oder Diisopropylstearat, 0,2 bis 0,5 g Polyvinylpyrrolidon (PVP), 5 bis 15 g N-Methyl-2-pyrrolidon, 0,5 bis 1,5 g 1-Lauryl-2-pyrrolidon, 65 bis 75 g Ethanol, 96 % und den Rest Wasser.

Das als Wirkstoff in der Arzneimittelzubereitung vorliegende Diclofenac kann in allen pharmazeutisch geeigneten Formen eingesetzt werden, z.B. als Diclofenac-Na, Diclofenac-Diethylamin, Diclofenac-Diethylammonium oder Diclofenac-Epolamin.

Die Erfindung wird durch die folgenden Beispiele weiter erläutert:

### Beispiel 1 - Herstellung einer Diclofenac-Lösung

Es wurden 100 g Lösung durch Zusammenmischen der folgenden Bestandteile hergestellt:

| | |
|---|---|
| Diclofenac-Natrium | 1,00 g |
| Dexpanthenol | 1,00 g |
| Diisopropyl-Adipat | 2,50 g |
| Polyvinylpyrrolidon (PVP) | 0,30 g |
| N-Methyl-2-pyrrolidon | 10,0 g |
| N-Dodecyl-2-pyrrolidon | 0,75 g |
| Ethanol 96 % | 70,0 g |
| HCl | 0,15 g |
| Wasser ad | 100,0 g |

### Beispiel 2 - In vitro Versuch an humaner Haut

Eine wie in Beispiel 1 hergestellte Diclofenac-Lösung, welche 1 % Diclofenac, berechnet als Natriumsalz, enthielt, wie sie zur therapeutischen Anwendung als Dosieraerosol gelangen kann, wurde hinsichtlich des Penetrationsvermögens mit einer herkömmlichen Voltaren® Emulgen-Formulierung verglichen.

### Hautpräparierung:

Weibliche Brust- und Bauchhaut wurde eingefroren, bei -20 °C gelagert und vor dem Verarbeiten aufgetaut. Nach Entfernen des subkutanen Fettes wurden die präparierten Hauptmembranen mit vollständiger Dicke auf Glasdiffusionszellen angeordnet.

### Diffusionszellen:

Die präparierten Hautmembranen wurden zwischen zwei Hälften einer horizontalen Glasdiffusionszelle des Typs Franz eingebracht, wobei das Stratum corneum in Richtung der Donorkammer angeordnet war. Die Zellen waren so ausgelegt, dass die zur Diffusion verfügbare Fläche etwa 1,0 cm² betrug, wobei die genaue Fläche für jede Diffusionszelle gemessen wurde. Auch das Rezeptorkammervolumen wurde für jede Zelle einzeln bestimmt.

Die Diffusionszellen wurden in ein Wasserbad mit konstanter Temperatur eingetaucht, sodass die Rezeptorkammern bei 37,0 ± 0,5 °C während des Experiments gehalten wurden. Dadurch wurde sichergestellt, dass die Hautoberflächentemperatur bei 32,0 ± 1 °C gehalten wurde. Die Rezeptorkammern der Diffusionszellen wurden mit einem bekannten Volumen eines Phosphatpuffers, pH 7,4 gefüllt, verschlossen und auf die gewünschte Temperatur gebracht. Für jede Testformulierung wurden 9 Versuche durchgeführt zusammen mit zwei Kontrollzellen, die unbehandelt waren.

### Aufbringen der Formulierungen:

Ein Glasstab wurde gewogen und die jeweilige Formulierung wurde auf das Ende des Glasstabes aufgebracht. Die Formulierung wurde dann mit dem Glasstab gleichmäßig über die Stratum corneum Oberfläche verteilt und der Glasstab rückgewogen, um die genaue Menge der auf die Hautoberfläche aufgebrachten Formulierung zu bestimmen. Die aufzutragende Menge betrug 10 mg/cm². Die Auftragungszeit stellte die Zeit 0 für den Versuch dar.

### Bestimmung der Permeation:

Nach der Auftragung der Formulierungen wurden 200 *µ*l Proben des Rezeptormediums nach 2, 4, 8, 24 und 48 Stunden unter Verwendung einer Mikropipette entnommen. Die Rezeptorkammern wurden mit 200 *µ*l frischem, auf die entsprechende Temperatur eingestelltem Rezeptormedium befüllt und die Proben wurden mit HPLC (Hochleistungsflüssigkeitschromatogrpahie) auf ihren Diclofenac-Gehalt analysiert.

### Ergebnisse:

Die Ergebnisse der Messungen (in *µ*g/cm²; arithmetisches Mittel ± Standardabweichung) sind in Tabelle 1 dargestellt:

**Tabelle 1**

| Zeit (Stunden) | 8 | 24 | 48 |
|---|---|---|---|
| Voltaren | 0,00 | 0,17 ± 0,05 | 0,80 ± 0,19 |
| erfindungsgemäße Diclofenac-Lösung | 0,01 ± 0,01 | 1,12 ± 0,54 | 4,53 ± 2,03 |

Die Versuche ergeben, dass sich in vitro nach 8 Stunden erste Konzentrationsunterschiede messen lassen, die sich nach 24 und 48 Stunden verstärken. Aus der Konzentrations-Zeitkurve der humanpharmakologischen in vitro Studie lässt sich eine theoretische Penetrationsverstärkung um den Faktor 5 gegenüber dem Standardpräparat ableiten.

### Beispiel 3 - In vivo Versuch an der Ratte

Es wurden die Wirkungen einer 1 %-igen Diclofenac-Lösung und eines 1 %-igen Voltaren-Gels verglichen. Dazu wurde in einer 14-tägigen dermalen Toxizitätsstudie Diclofenac täglich an Ratten beiderlei Geschlechts in Dosiseinheiten von 0,1 g/Tag epikutan verabreicht. Die Studie umfasste jeweils 12 männliche und 12 weibliche Tiere in jeder Gruppe. Zur Überprüfung der Absorption wurden Blutproben nach 4 Stunden der Verabreichung an den Testtagen 1, 3, 7 und 14 entnommen.

Die Gegenwart von mit 0,1 g/Tier dermal aufgebrachtem Diclofenac wurde im Plasma der Weibchen der beiden Gruppen bestimmt. Die durchschnittlichen Plasmakonzentrationen während der Behandlung betrugen 0,164 *µ*g/ml für die mit der erfindungsgemäßen Lösung behandelten Tiere, sowie 0,332 *µ*g/ml für die mit Voltaren Emulgel behandelten Tiere. Bei den weiblichen Tieren weisen die toxikokinetischen Daten nach Anwendung des Standardpräparates somit doppelt höhere Plasmaspiegel auf als nach Anwendung der erfindungsgemäßen Lösung. Die Gegenwart von Diclofenac im Plasma der männlichen Tiere konnte nicht nachgewiesen werden, da die Konzentration unterhalb des Bestimmungsgrenzwertes (0,05 *µ*g/ml) lag.

Zusammenfassend kann festgehalten werden, dass sich nach 14-tägiger dermaler Anwendung der erfindungsgemäßen Lösung gegenüber dem Standardpräparat Voltaren in der Verträglichkeit und dem Toxizitätsprofil keine wesentlichen Unterschiede aufzeigen lassen, welche auf eine höhere systemische Verfügbarkeit der erfindungsgemäßen Lösung hinweisen würden, obwohl (vergleiche Beispiel 2) eine 5-fach höhere Penetration nachgewiesen wurde.

### Beispiel 4 - Klinische Wirkung

Es wurde ein multizentrischer klinischer Versuch durchgeführt, bei dem eine 1 %-ige Diclofenac-Lösung als Dosieraerosol mit einem 1 %-igen Voltaren® Emulgen bei der Behandlung von Osteoarthritis verglichen wurde. Es handelte sich dabei um eine "single blind, randomised, multiple dose, multicenter safety and efficacy comparison of two topical formulations of 1,0 % Diclofenac (Assaren® Metered Dose Pumpspray vs Voltaren® Emulgel) in the treatment of gonarthritis". Dazu wurden 120 Patienten beiderlei Geschlechts mit mäßigen bis ernsten Kniebeschwerden aufgrund von Osteoarthritis untersucht. Die Formulierungen wurden jeweils 4-mal täglich auf das Knie auf eine Fläche von ≤ 200 cm² aufgebracht. Dabei wurde eine therapeutische Äquivalenz von 88 mg der Diclofenac-Lösung mit 120 mg des Voltaren-Gels als Standard festgestellt. Weiterhin bestätigte der multizentrische klinische Versuch, dass die erfindungsgemäße Lösung bei Patienten gut verträglich ist und keine Toxizität bewirkte. Der therapeutische Vorteil der geringeren Wirkstoffdosis ist vor allem auf die Zusammensetzung der erfindungsgemäßen Arzneimittelzubereitung zurückzuführen.

## Patentansprüche

1. Arzneimittelzubereitung zur topischen Anwendung, enthaltend Diclofenac als Wirkstoff,
**dadurch gekennzeichnet,**
**dass** der Wirkstoff in einem Lösungsmittelgemisch gelöst vorliegt, welches mindestens einen Alkylalkohol mit 2 bis 4 C-Atomen als Hauptbestandteil, mindestens ein kurzkettiges N-Alkylpyrrolidon mit 1 bis 4 C-Atomen im Alkylrest und mindestens ein mit einem langkettigen Alkylrest, der 10 bis 16 C-Atome aufweist, substituiertes Pyrrolidon enthält.

2. Zubereitung nach Anspruch 1,
**dadurch gekennzeichnet,**
**dass** der Wirkstoffgehalt 0,5 bis 5 % Gewicht pro Volumen beträgt.

3. Zubereitung nach einem der vorhergehenden Ansprüche,
**dadurch gekennzeichnet,**
**dass** das kurzkettige N-Alkylpyrrolidon zum langkettigen Alkylpyrrolidon im Molverhältnis 10 - 100:1 vorliegt.

4. Zubereitung nach einem der vorhergehenden Ansprüche
**dadurch gekennzeichnet,**
**dass** das Molverhältnis der Pyrrolidone 20 - 40:1 beträgt.

5. Zubereitung nach einem der vorhergehenden Ansprüche,
**dadurch gekennzeichnet,**
**dass** der Alkylalkohol Ethanol ist.

6. Zubereitung nach einem der vorhergehenden Ansprüche,
**dadurch gekennzeichnet,**
**dass** der Alkoholgehalt 50 bis 80 Gew.-% beträgt.

7. Zubereitung nach einem der vorhergehenden Ansprüche,
**dadurch gekennzeichnet,**
**dass** sie 65 bis 75 Gew.-% Ethanol, 5 bis 20 Gew.-% N-Methylpyrrolidon und 0,3 bis 2 Gew.-% 1-Lauryl-2-pyrrolidon enthält.

8. Zubereitung nach einem der vorhergehenden Ansprüche,
**dadurch gekennzeichnet,**
**dass** sie weiterhin pharmazeutisch geeignete Hilfs- oder/und Trägerstoffe umfasst.

9. Zubereitung nach einem der vorhergehenden Ansprüche,
**dadurch gekennzeichnet,**
**dass** sie 0,5 bis 5 Gew.-% Diclofenac, 65 bis 75 Gew.-% Ethanol, 5 bis 20 Gew.% Methylpyrrolidon, 0,3 bis 2 Gew.-% 1-Lauryl-2-pyrrolidon, 1,0 bis 5,0 Gew.% Dexpanthenol, 1 bis 5 Gew.-% Diisopropyl-adipat oder/und Diisopropyl-palmitat oder/und Diisopropyl-stearat, 0,1 bis 3 Gew.-% Polyvinylpyrrolidon und als Rest Wasser enthält.

## Claims

1. A medicinal preparation for topical application, containing diclofenac as active ingredient, **characterised in that** the active ingredient is present dissolved in a solvent mixture which contains at least one alkyl alcohol having 2 to 4 carbon atoms as main constituent, at least one short-chain N-alkyl pyrrolidona having 1 to 4 carbon atoms in the alkyl radical and at least one pyrrolidone substituted with a long-chain alkyl radical which has 10 to 16 carbon atoms.

2. A preparation according to Claim 1, **characterised in that** the active ingredient content is 0.5 to 5 % by weight by volume.

3. A preparation according to any one of the preceding Claims, **characterised in that** the short-chain N-alkyl pyrrolidone is present in a molar ratio of 10 - 100:1 in relation to the long-chain alkyl pyrrolidone.

4. A preparation according to any one of the preceding Claims, **characterised in that** the molar ratio of the pyrrolidone is 20 - 40:1.

5. A preparation according to any one of the preceding Claims, **characterised in that** the alkyl alcohol is ethanol.

6. A preparation according to any one of the preceding Claims, **characterised in that** the alcohol content is 50 to 80 % by weight.

7. A preparation according to any one of the preceding Claims, **characterised in that** it contains 65 to 75 % by weight of ethanol, 5 to 20 % by weight of N-metbyl pyrrolidone and 0.3 to 2 % by weight of 1-lauryl-2-pyrrolidone.

8. A preparation according to any one of the preceding Claims, **characterised in that** it further comprises pharmaceutically suitable adjuvants and/or supports.

9. A preparation according to any one of the preceding Claims, **characterised in that** it contains 0.5 to 5 % by weight of diclofenac, 65 to 75 % by weight of ethanol, 5 to 20 % by weight of methyl pyrrolidone, 0.3 to 2 % by weight of 1-lauryl-2-pyrrolidone, 1.0 to 5.0 % by weight of dexpanthenol, 1 to 5 % by weight of diisopropyl-adipate and/or of diisopropyl-palmitate and/or of diisopropyl-stearate, 0.1 to 3 % by weight of polyvinyl pyrrolidone and the remainder water.

## Revendications

1. Préparation médicamenteuse pour l'application topique, contenant du diclofénac en tant que principe actif,
**caractérisée en ce que**,
le principe actif se présente dissous dans un mélange de solvant qui contient au moins un alcool d'alkyle avec 2 à 4 atomes C comme composant principal, au moins une N-alkylpyrrolidone à chaîne courte avec 1 à 4 atomes C dans le reste alkyle et au moins une pyrrolidone substituée avec un reste alkyle à chaîne longue qui présente 10 à 16 atomes C.

2. Préparation selon la revendication 1,
**caractérisée en ce que**,
la teneur en principe actif est de 0,5 à 5 % en volume.

3. Préparation selon la revendication 1,
**caractérisée en ce que**,
le rapport molaire entre la N-alkylpyrrolidone à chaîne courte et l'alkylpyrrolidone à chaîne longue est 10-100:1.

4. Préparation selon l'une des revendications précédentes,
**caractérisée en ce que**,
le rapport mùolaire entre les pyrrolidones est de 20-40:1.

5. Préparation selon l'une des revendications précédentes,
**caractérisée en ce que**,
l'alcool d'alkyle est l'éthanol.

6. Préparation selon l'une des revendications précédentes,
**caractérisée en ce que**,
la teneur en alcool est de 50 à 80% en poids.

7. Préparation selon l'une des revendications précédentes,
**caractérisée en ce que**,
elle contient 65 à 75 % en poids d'éthanol, 5 à 20 % en poids de N-méthylpyrrolidone et 0,3 à 2 % en poids de 1-lauryl-2-pyrrolidone.

8. Préparation selon l'une des revendications précédentes,
**caractérisée en ce que**,
elle renferme de plus des adjuvants ou/et véhicules pharmaceutiquement appropriés.

9. Préparation selon l'une des revendications précédentes,
**caractérisée en ce que**,
elle contient 0,5 à 5 % en poids de diclofénac, 65 à 75 % en poids d'éthanol, 5 à 20 % en poids de méthylpyrrolidone, 0,3 à 2 % en poids de 1-lauryl-2-pyrrolidone, 1,0 à 5,0 % en poids de dexpanthénol, 1 à 5 % en poids d'adipate de diisopropyle ou/et palmirate de diisopropyle ou/et de stéarate de diisopropyle, 0,1 à 3 % en poids de polyvinylpyrrolidone et de l'eau pour le reste.
